Europäisches Patentamt

European Patent Office

Office européen des brevets

(19)

(11) Publication number: **0 371 421 B1**

# (12) EUROPEAN PATENT SPECIFICATION

(45) Date of publication of patent specification: **21.12.94**  (51) Int. Cl.[5]: **C08F 220/06**, A61K 9/20

(21) Application number: **89121813.3**

(22) Date of filing: **25.11.89**

The file contains technical information submitted after the application was filed and not included in this specification

(54) **Crosslinked polyacrylic acid.**

(30) Priority: **28.11.88 US 276839**

(43) Date of publication of application:
**06.06.90 Bulletin 90/23**

(45) Publication of the grant of the patent:
**21.12.94 Bulletin 94/51**

(84) Designated Contracting States:
**AT BE CH DE ES FR GB GR IT LI LU NL SE**

(56) References cited:
**US-A- 4 615 697**
**US-A- 4 758 641**

**JOURNAL OF PHARMACEUTICAL SCIENCES, vol. 74, no. 4, April 1985, pages 399-504,American Pharmaceutical Association; HUNG SENG CH'NG et al.: "Bioadhesivepolymers as platforms for oral controlled drug delivery II: Synthesis andevaluation of some swelling, water-insoluble bioadhesive polymers"**

(73) Proprietor: **The B.F. Goodrich Company**
**3925 Embassy Parkway**
**Akron Ohio 44313 (US)**

(72) Inventor: **Sehm, Eugene Joseph**
**1104 Portage Lakes Drive**
**Akron Ohio 44319 (US)**

(74) Representative: **von Kreisler, Alek,**
**Dipl.-Chem. et al**
**Patentanwälte**
**von Kreisler-Selting-Werner**
**Postfach 10 22 41**
**D-50462 Köln (DE)**

**Description**

Background of the Invention

Polymers of unsaturated carboxylic acids and salts thereof are well known. These polymers include homopolymers and copolymers which contain up to 10 weight percent of other copolymerizable monomers. Typical monomers include acrylic acid, methacrylic acid, maleic acid or its anhydride, itaconic acid, and the like. U.S. patent 2,798,053, for instance, discloses copolymers of acrylic acid with small amounts of polyalkenyl polyether crosslinkers which are gel-like and, especially in the form of their salts, can absorb large quantities of water or solvents with subsequent substantial increase in volume. U.S. patents 3,940,351 and 4,062,817 describe polymers of an unsaturated carboxylic acid and at least one acrylic or methacrylic ester wherein the alkyl groups contain 1 to 30 carbon atoms. Such polymers are also effective thickening agents, even in the presence of substantial amounts of inorganic salts. U.S. patents 3,915,921 and 4,066,583 disclose preparation of same or similar polymers in similar systems.

U.S. patent 4,267,103 discloses polymers of unsaturated carboxylic acids or salts thereof in certain solvents wherein more than 1% by weight of the carboxyl groups are neutralized. Such polymers have molecular weight greater than 500 and up to several million, but generally, in the range of 10,000 to one million. Such polymers are also effective thickening agents.

USP 4,758,641 discloses polymerization of acrylic acid in a solvent selected from acetone, alkyl acetates, and mixtures thereof. Polymerization is carried out in the presence of an effective amount of an initiator and crosslinker and some of the carboxyl groups on the acrylic acid are neutralized.

Polycarbophil is defined as polyacrylic acid crosslinked with divinyl glycol. Polycarbophil, therefore, is a free acid polymer which has numerous applications due to its capacity to absorb fluids, such as water, and its bioadhesive property of being able to adhere to a mucous membrane in the eyes, nose, mouth, gastrointestinal tract, vaginal cavity and rectal canal. A salt of Polycarbophil, such as calcium Polycarbophil, does not have the bioadhesive property and is not used in applications where the material would be expected to attach itself.

The Journal of Pharmaceutical Science, Vol. 74, no. 4, pages 399-405 discloses the polymerization of acrylic acid monomer in a concentrated aqueous salt solution, such as magnesium sulfate, or another water soluble nonredox multivalent inorganic salt, in the presence of an initiator and the divinyl glycol crosslinker. The resulting polymer was washed with water several times whereupon it swelled to many times its volume, to remove the salt therefrom. After washing with water, the swollen polymer was dried until a solid chunk of the polymer was obtained, which was only a fraction of its swollen size. The dried polymer was then ground to the desired particle size and used in applications where its swelling property and/or its bioadhesive property were needed.

To reduce the drying cost, the polymer, after its formation, was treated with calcium carbonate or calcium hydroxide whereby the swollen polymer was collapsed upon formation of the calcium Polycarbophil. The calcium Polycarbophil was dried to a solid chunk and then ground to the desired particle size. Whereas Polycarbophil has swelling and bioadhesive properties that make it difficult to pass through the wet mouth, calcium Polycarbophil is ideally suited for oral dose form as it does not swell or become slimey in the mouth. The free acid polymer is liberated in the acid stomach from the calcium Polycarbophil.

Summary of the Invention

Acrylic acid monomer is polymerized in a non-aqueous solvent selected from acetone and alkyl acetate of 1 to 6 carbon atoms in the alkyl group and in the presence of an initiator and divinyl glycol crosslinker whereby the polymer is obtained in particulate form of less than 10 micronmeter average particle size without grinding which gives mucilage viscosity in excess of 50,000 mPa•s (cps) when measured in 1% concentration in water. This preparation process avoids the expensive drying and grinding and retains the swelling, small particle size and bioadhesivity attributes of Polycarbophil.

Detailed Description of the Invention

This invention pertains to Polycarbophil and salts thereof and to preparation thereof in a solvent selected from acetone and alkyl acetates containing 1 to 6 carbon atoms in the alkyl group, said process comprises polymerizing acrylic acid monomer in a solvent selected from acetone, alkyl acetates of 1 to 6 carbon atoms in the alkyl group, and mixtures thereof, in the presence of divinyl glycol (3,4-dihydroxy-1,5-hexadiene) crosslinker and an initiator.

Polycarbophil and alkali metal and alkaline earth metal salts thereof, prepared in this manner, have the attributes of bioadhesivity, small particle size without grinding and viscosity of above 50,000 mPa•s (cps) when 1% by weight mucilages thereof are measured in water. It is estimated that weight average molecular weight of Polycarbophil or crosslinked polyacrylic acid is in the range of 100,000 to 10 million, preferably one half million to 5 million.

The polyacrylic acid of this invention is a water-insoluble, cross linked carboxy-functional polymer that contains specified amounts of carboxyl functionality and crosslinking agent. In addition, the polymer of this invention is also a useful bioadhesive which exhibits adhesion between two pieces of freshly excised rabbit stomach tissue of at least $5 \cdot 10^{-4}$ N/cm$^2$ (50 dynes/cm$^2$) when measured in the manner described in USP 4,615,697.

As already pointed out, Polycarbophil is described as being polyacrylic acid crosslinked with divinyl glycol. Suitable monomer herein, therefore, is acrylic acid and salts thereof.

To prevent gelling of the polymer and to promote discrete particle formation during polymerization, at least a part of the carboxyl groups should be neutralized with a group I-A metal compound in the form of a hydroxide, oxide or carbonate. Examples of these include sodium or potassium, as well as reaction with ammonia and certain amines including morpholine, mono, di and triethanolamine, mono propanolamine, and other amines where the partial polymeric salt is less soluble in the reaction medium.

Preferably greater than 0.1% by weight of the carboxyl groups on the monomer are neutralized or formed into a salt of the above listed materials. More preferably, 1% by weight to 10% by weight of the carboxyl groups are neutralized or converted to the equivalent salt prior to polymerization, especially 1% to 5%.

Normally, polar and medium to strongly hydrogen bonded solvents are not suitable as solvents for carboxyl containing polymers free of the salts because they swell the free acid containing polymers to gels, which is undesirable.

The solvents used according to the invention are liquid at room temperature of about 22°C and are selected from acetone and lower alkyl acetates containing 1 to 6, preferably 2 to 4, carbon atoms in the alkyl group. Specific examples of such acetates include ethyl acetate, isopropyl acetate or, n-butyl acetate. Preferred solvent is ethyl acetate. Amount of the solvent used should be such that the monomer solids content is up to 30% by weight, preferably 10 to 20%. With the solvents enumerated herein, there is no need for washing with water to remove magnesium sulfate, or another salt which is soluble in water. The solvents are removed by evaporation or by drying. In this way, water-washings are avoided and the problems associated with swelling of the acrylic acid polymer when contacted with water is also absent since in solvents described herein, the product does not swell nearly as much.

It was unexpectedly found that polyacrylic acid partially cross linked with divinyl glycol prepared in different solvents in the manner described herein did not yield 1% mucilages with viscosity exceeding 50,000 mPa•s (cps) at pH 7.2-7.8 at 20 rpm having particle size less than 10 micrometer. Results of these experiments are given below in Table I:

Table I

| Polymerization Medium | Average Particle Size | Brookfield Viscosity, mPa•s (cps) |
|---|---|---|
| Ethyl Acetate | < 10 $\mu$m | 59,200 |
| Methylene Chloride | < 10 $\mu$m | 1,000 |
| Benzene | < 10 $\mu$m | 1,730 |
| 35% MgSO$_4$ | < 12 $\mu$m | 628 |

The polyacrylic acid made in 35% aqueous solution of magnesium sulfate was ground to the average particle size of less than about 12 micrometer. The other acrylic acid polymers all had average particle size of less than about 10 micrometer as prepared without grinding.

As is apparent from Table I, above, only polymerization of acrylic acid in ethyl acetate yielded a polymer which, in the form of a 1% mucilage, gave viscosity in excess of 50,000 mPa•s (cps).

Amount of water in the solvent should be as low as possible since if water is allowed to exceed 3% in the solvent, the reaction mass becomes a solid, rubbery mass, which is undesirable. Desirable results can be achieved by continuously removing water from the solvent as by passing the solvent through a distillation column or through a bed of a desiccant or a substance which will remove water from the solvent. This problem is compounded by the fact that the polymerization produces water as a by-product. However, water can be removed and amount thereof in the reaction mass can be controlled to a level below 3%, preferably 0.05 to 1%, in the solvent, in the manner described above.

Polymerization of the acrylic acid monomer or its salt in the solvent medium is usually carried out in the presence of a free radical initiator in a closed vessel in an inert atmosphere and under autogenous pressure, artificially-induced pressure, or in an open vessel under reflux at atmospheric pressure. Temperature of the polymerization may be varied up to 100°C, preferably 40 to 80°C, depending on the type of initiator selected. Suitable free radical initiators are those which will convert essentially all of the monomer to polymer at the reaction temperature. Examples of such free radical initiators include di(2-ethylhexyl) peroxydicarbonate, di(sec-butyl) peroxydicarbonate, di(isopropyl) peroxydicarbonate, dicyclohexyl peroxydicarbonate, dicetyl peroxydicarbonate, di(n-propyl) peroxydicarbonate, lauroyl peroxide, and other like peroxides and peroxydicarbonates. The di(2-ethylhexyl) peroxydicarbonate is effective at a reaction temperature of 45 to 55°C whereas lauroyl peroxide is effective at a reaction temperature of 70 to 80°C. Amount of the initiator is generally less than 5%, preferably 0.05 to 2.0%, and especially 0.1 to 1% by weight based on the weight of the monomer charge.

The crosslinker for making Polycarbophil is divinyl glycol or 3,4-dihydroxy-1,5-hexadiene. Amount of the crosslinker per 100 weight parts monomer can vary up to 5% by weight, preferably 0.01 to 3%, especially 0.5 to 2% by weight.

It was not apparent, based on the known prior art, that preparation of acrylic acid homopolymer in ethyl acetate with divinyl glycol crosslinker would result in a polymer which would have sufficient viscosity in water. This conclusion is based on the fact that the prior art did not provide any guidance as to what could be expected in terms of obtaining viscosity in excess of about 50,000 mPa•s (cps) with divinyl glycol crosslinker in a specific solvent. The following Table II summarizes results of polymerization of acrylic acid in an identical manner in different solvents and with different crosslinkers, wherein "NO" indicates that viscosity in excess of about 50,000 mPa•s (cps) was not attained when measured as a 1% concentration of the polymer in water and "YES" indicates that such viscosity was attained:

**Table II**

| Crosslinker | Solvent | | | | |
|---|---|---|---|---|---|
| | $MgSO_4/H_2O$ | $CH_2Cl_2$ | $C_6H_6$ | EtAc/CH | EtAc |
| Allyl Sucrose (AS) | NO | YES | YES | YES | YES |
| Allyl Pentaeritol (APE) | NO | YES | YES | YES | YES |
| Divinyl Glycol (DVG) | YES | NO | NO | NO | YES |

The $MgSO_4/H_2O$ solvent was a saturated aqueous solution of magnesium sulfate or about 35% by weight of magnesium sulfate salt in water; the $CH_2Cl_2$ solvent was methylene chloride; the $C_6H_6$ solvent was benzene; the EtAc/CH solvent was an azeotrope mixture of ethyl acetate and cyclohexane or about 54/46 ethyl acetate to cyclohexane on weight bases; and the EtAc solvent was ethyl acetate.

On the basis of the data in Table II, above, it was not possible for a person skilled in this art to predict what the expectation might be with respect to viscosity of polyacrylic acid prepared with divinyl glycol crosslinker. Preponderance of the evidence indicates that with divinyl glycol crosslinker in ethyl acetate, viscosity would be below 50,000 mPa•s (cps) when measured at 1% concentration of the polymer in water. A contrary result, however, was obtained.

Preparation of calcium Polycarbophil or another salt of Polycarbophil can be made after Polycarbophil is made. This can be accomplished by treating Polycarbophil with a material such as calcium carbonate or calcium hydroxide to convert Polycarbophil to a salt thereof, such as calcium Polycarbophil.

The products of this invention have exceptional bioadhesive properties. The small particle size of the products herein lends itself to better adhesion to mucous membranes and to more extensive applications in non-tablet areas such as lotions, suspensions, gels or syrups. Mix smoothness is exceptionally good and useful in drug and cosmetic applications.

The invention will now be illustrated with a specific example of preparing Polycarbophil or polyacrylic acid cross linked with a small amount of divinyl glycol. The polyacrylic acid product has bioadhesive property and a particle size of less than 10 micrometer without grinding and its 1% aqueous solution has viscosity exceeding 50,000 mPa•s (cps).

# EP 0 371 421 B1

Example

The following ingredients were used in amounts indicated to prepare Polycarbophil:

| | |
|---|---|
| Ethyl Acetate Solvent | 1316.42 grams |
| Acrylic Acid Monomer | 180.00 grams |
| Potassium Carbonate | 2.59 grams |
| Divinyl Glycol Crosslinkers | 0.09 grams |
| EHP Initiator | 0.90 grams |
| | 1500.00 grams |

The EHP Initiator was di(2-ethylhexyl) peroxydicarbonate.

A 2-liter jacketed reactor was used in this preparation which reactor had cooling capability and which was provided with a reflux condenser. The reactor was purged with nitrogen to remove moisture and to maintain an inert atmosphere in the reactor. The acrylic acid was preneutralized with anhydrous potassium carbonate by mixing potassium carbonate in acrylic acid until potassium carbonate dissolved in acrylic acid, which took about one-quarter of an hour, while the nitrogen purge was continued. The neutralized acrylic acid was charged to the purged reactor followed by ethyl acetate. The crosslinker was prepared in a 6-dram (10.626 g) vile in ethyl acetate and then charged to the reactor. The solution in the reactor was agitated for a few minutes and then agitation was stopped and the nitrogen purge was placed at bottom of reactor and the contents of the reactor were purged with nitrogen for about 20 minutes. With the nitrogen purge at top of reactor again, the initiator was charged to reactor and heating of the reactor was commenced and was continued for 6 hours.

The polymer recovered from the reactor was rotary vacuum dried to remove remaining ethyl acetate solvent. The polymer was particulate and free-flowing with average particle size of less than 10 micrometers and its Brookfield viscosity of 1% mucilage in water was 59,200 mPa•s (cps) measured at 20 rpm. The pH of the mucilage was about 7.5. It was also determined that the polymer had the bioadhesive property.

## Claims

1. Process for preparing crosslinked polyacrylic acid which has particle size of less than 10 micrometer without grinding and which has Brookfield viscosity of greater than 50,000 mPa•s (cps) in the form of a 1% by weight thereof in water, said process comprising polymerizing acrylic acid monomer in a solvent selected from acetone, alkyl acetates of 1 to 6 carbon atoms in the alkyl group, and mixtures thereof, in the presence of divinyl glycol (3,4-dihydroxy-1,5-hexadiene) crosslinker and an initiator.

2. Process of claim 1 wherein the process includes the step of neutralizing up to 10% of carboxyl of the acrylic acid groups; and wherein the solvent contains less than 3% water.

3. Process of claim 2 wherein the solvent is selected from alkyl acetates having 2 to 4 carbons in the alkyl group, and mixtures thereof, wherein amount of the initiator is 0.05 to 2%, based on the weight of the monomer; and wherein amount of the crosslinker is 0.01 to 3%, based on the weight of the monomer.

4. Process of claim 3 wherein the solvent is ethyl acetate and the crosslinked polyacrylic acid has bioadhesive property.

5. Process of claim 4 wherein the initiator is selected from di(2-ethylhexyl) peroxydicarbonate, di(sec-butyl) peroxydicarbonate, di(n-propyl) peroxydicarbonate, lauroyl peroxide, and mixtures thereof; and wherein the polyacrylic acid is not washed with water immediately after the acrylic acid monomer is polymerized.

6. Process of claim 5 wherein the monomer concentration in the ethyl acetate solvent is 10-20% by weight, wherein amount of the crosslinker is 0.5 to 2% by weight; wherein amount of the initiator is 0.1 to 1% by weight; and wherein 1 to 5% of the carboxyl groups in the acrylic acid is neutralized.

7. Crosslinked acrylic acid polymer which has particle size of less than 10 micrometer as obtained without grinding and which polymer has Brookfield viscosity of greater than 50,000 mPa•s (cps) when measured in water at 1% by weight concentration obtainable by polymerizing acrylic acid monomer in a solvent selected from acetone, alkyl acetates of 1 to 6 carbon atoms in the alkyl group, and mixtures thereof, in the presence of divinyl glycol (3,4-dihydroxy-1,5-hexadiene) crosslinker and an initiator.

8. Polymer of claim 7 which has bioadhesive property with respect to a mucous membrane.

9. Polymer of claim 8 which has bioadhesive property of at least $5 \cdot 10^{-4}$ N/cm$^2$ (50 dynes/cm$^2$) when adhesion is measured between two pieces of freshly excised rabbit stomach tissue.

10. Polymer of claim 9 prepared in presence of ethyl acetate solvent and in the presence of divinyl glycol crosslinker.

**Patentansprüche**

1. Verfahren zur Herstellung vernetzter Polyacrylsäure, die ohne Vermahlen eine Teilchengröße von Weniger als 10 $\mu$m hat und die in Form von 1 Gew.-% derselben in Wasser eine Brookfield-Viskosität von mehr als 50 000 mPa•s (cP) hat, wobei dieses Verfahren das Polymerisieren von Acrylsäure-Monomer in einem aus Aceton, Alkylacetaten mit 1 bis 6 Kohlenstoff-Atomen in der Alkyl-Gruppe und deren Mischungen ausgewählten Lösungsmittel in Gegenwart von Divinylglycol[3,4-Dihydroxy-1,5-hexadien]-Vernetzungsmittel und einem Initiator umfaßt.

2. Verfahren nach Anspruch 1, wobei das Verfahren den Schritt des Neutralisierens von bis zu 10 % des Carboxyls der Acrylsäure-Gruppen einschließt und das Lösungsmittel weniger als 3 % Wasser enthält.

3. Verfahren nach Anspruch 2, worin das Lösungsmittel aus Alkylacetaten mit 2 bis 4 Kohlenstoff-Atomen in der Alkyl-Gruppe und deren Mischungen ausgewählt ist, worin die Menge des Initiators 0,05 bis 2 %, bezogen auf das Gewicht des Monomers, beträgt und worin die Menge des Vernetzungsmittels 0,01 bis 3 %, bezogen auf das Gewicht des Monomers, beträgt.

4. Verfahren nach Anspruch 3, worin das Lösungsmittel Ethylacetat ist und die vernetzte Polyacrylsäure Bioklebeeigenschaften besitzt.

5. Verfahren nach Anspruch 4, worin der Initiator aus Di(2-ethylhexyl)peroxydicarbonat, Di(sec-butylperoxydicarbonat, Di(n-propyl)peroxydicarbonat, Lauroylperoxid und deren Mischungen ausgewählt ist und worin die Polyacrylsäure unmittelbar nach dem Polymerisieren des Acrylsäure-Monomers nicht mit Wasser gewaschen wird.

6. Verfahren nach Anspruch 5, worin die Monomer-Konzentration in dem Ethylacetat-Lösungsmittel 10 bis 20 Gew.-% beträgt, worin die Menge des Vernetzungsmittels 0,5 bis 2 Gew.-% beträgt, worin Menge des Initiators 0,1 bis 1 Gew.-% beträgt und worin 1 bis 5 % der Carboxyl-Gruppen in der Acrylsäure neutralisiert sind.

7. Vernetztes Acrylsäure-Polymer, das eine Teilchengröße von weniger als 10 $\mu$m, erhalten ohne Vermahlen, hat und das, gemessen in Wasser bei einer Konzentration von 1 Gew.-%, eine Brookfield-Viskosität von mehr als 50 000 mPa•s (cP) hat, erhältlich durch Polymerisieren von Acrylsäure-Monomer in einem aus Aceton, Alkylacetaten mit 1 bis 6 Kohlenstoff-Atomen in der Alkyl-Gruppe und deren Mischungen ausgewählten Lösungsmittel in Gegenwart von Divinylglycol[3,4-Dihydroxy-1,5-hexadien]-Vernetzungsmittel und einem Initiator.

8. Polymer nach Anspruch 7, das Bioklebeeigenschaften in bezug auf eine Schleimhaut besitzt.

9. Polymer nach Anspruch 8, das Bioklebeeigenschaften von wenigstens $5 \cdot 10^{-4}$ N/cm$^2$ (50 dyn/cm$^2$) hat, wenn die Haftkraft zwischen zwei Stücken von frisch herausgeschnittenem Kaninchen-Magengewebe gemessen wird.

10. Polymer nach Anspruch 9, hergestellt in Gegenwart von Ethylacetat-Lösungsmittel und in Gegenwart von Divinylglycol-Vernetzungsmittel.

**Revendications**

1. Procédé de préparation d'acide polyacrylique réticulé ayant une taille particulaire inférieure à 10 micromètres sans broyage et ayant une viscosité Brookfield supérieure à 50 000 mPa.s (cps) sous la forme d'une solution à 1% en poids dans l'eau, ledit procédé comprenant la polymérisation d'un monomère d'acide acrylique dans un solvant choisi parmi l'acétone, les acétates d'alkyle ayant de 1 à 6 atomes de carbone dans le groupe alkyle, et leurs mélanges, en présence d'un agent réticulant divinyl glycol (3,4-dihydroxy-1,5-hexadiène) et d'un inducteur.

2. Procédé de la revendication 1 où le procédé inclut l'étape de neutralisation d'une quantité allant jusqu'à 10% de carboxyle des groupes acide acrylique ; et où le solvant contient moins de 3% d'eau.

3. Procédé de la revendication 2 dans lequel le solvant est choisi parmi les acétates d'alkyle ayant de 2 à 4 atomes de carbone dans le groupe alkyle, et leurs mélanges, où la quantité d'inducteur est de 0,05 à 2%, sur la base du poids du monomère ; et où la quantité d'agent réticulant est de 0,01 à 3%, sur la base du poids du monomère.

4. Procédé de la revendication 3, dans lequel le solvant est l'acétate d'éthyle et l'acide polyacrylique réticulé a une propriété de bioadhérence.

5. Procédé de la revendication 4, dans lequel l'inducteur est choisi parmi le peroxydicarbonate de di(2-éthylhexyle), le peroxydicarbonate de di(sec-butyle), le peroxydicarbonate de di(n-propyle), le peroxyde de lauroyle, et leurs mélanges ; et où l'acide polyacrylique n'est pas lavé à l'eau immédiatement après polymérisation du monomère d'acide acrylique.

6. Procédé de la revendication 5 dans lequel la concentration de monomère dans le solvant acétate d'éthyle est de 10-20% en poids, où la quantité d'agent réticulant est de 0,5 à 2% en poids ; où la quantité d'inducteur est de 0,1 à 1% en poids ; et où de 1 à 5% des groupes carboxyle dans l'acide acrylique sont neutralisés.

7. Polymère d'acide acrylique réticulé ayant une taille particulaire inférieure à 10 micromètres tel qu'obtenu sans broyage, ce polymère ayant une viscosité Brookfield supérieure à 50 000 mPa.s(cps) mesurée dans l'eau à 1% de concentration pondérale que l'on peut obtenir en polymérisant le monomère d'acide acrylique dans un solvant choisi parmi l'acétone, les acétates d'alkyle ayant de 1 à 6 atomes de carbone dans le groupe alkyle, et leurs mélanges, en présence d'un agent réticulant divinyl glycol (3,4-dihydroxy-1,5-hexadiène) et d'un inducteur.

8. Polymère de la revendication 7 qui a une propriété de bioadhérence par rapport à une membrane muqueuse.

9. Polymère de la revendication 8 qui a une propriété de bioadhérence d'au moins $5 \times 10^{-4}$ N/cm$^2$ (50 dynes/cm$^2$) lorsque l'adhérence est mesurée entre deux morceaux de tissus d'estomac de lapin fraîchement excisé.

10. Polymère de la revendication 9 préparé en présence d'un solvant acétate d'éthyle et en présence d'un agent réticulant divinyl glycol.